# EUROPEAN PATENT SPECIFICATION

(11) **EP 3 890 592 B1**
(45) Date of publication and mention of the grant of the patent: **17.04.2024**
(21) Application number: 19893021.6
(22) Date of filing: 06.12.2019
(51) Int. Cl.: A61B 5/00, G10L 25/66, A61B 5/12, G16H 50/20

(54) **SPEECH DISCRIMINATION TEST SYSTEM**
TESTSYSTEM ZUR SPRACHUNTERSCHEIDUNG
SYSTÈME DE TEST DE DISCRIMINATION VOCALE

(30) Priority: 07.12.2018 AU 2018904679; 29.03.2019 AU 2019901071; 26.04.2019 AU 2019901407
(43) Date of publication of application: 13.10.2021
(73) Proprietor: Cochlear Limited, Macquarie University, NSW 2109 (AU)
(72) Inventor: DILLON, Harvey, Albert, Macquarie University, NSW 2109 (AU); CHING, Teresa, Yuk Ching, Macquarie University, NSW 2109 (AU); HOU, Sanna, Yuk Lan, Macquarie University, NSW 2109 (AU); CHONG-WHITE, Nicola, Raewyn, Macquarie University, NSW 2109 (AU)
(74) Representative: Grünecker Patent- und Rechtsanwälte PartG mbB
(86) International application number: PCT/IB2019/001317
(87) International publication number: WO 2020/115553

(56) References cited:
- WO-A1-2011/155197
- JP-A- H10 511 472
- US-A1- 2007 299 654
- US-A1- 2013 202 124
- US-A1- 2017 273 602
- US-A1- 2017 273 602
- US-A1- 2018 227 682
- US-B2- 9 148 732
- Finlay: "Teaching heart auscultation to health professionals" In: "Teaching heart auscultation to health professionals", 31 December 2011 (2011-12-31), XP055927461, * pages 68-69 *
- NICOLA BRUNSWICK ET AL: "Early cognitive profiles of emergent readers: A longitudinal study", JOURNAL OF EXPERIMENTAL CHILD PSYCHOLOGY, vol. 111, no. 2, 29 September 2011 (2011-09-29), pages 268-285, XP028125493, ISSN: 0022-0965, DOI: 10.1016/J.JECP.2011.08.001 [retrieved on 2011-08-10]

## Description

### FIELD OF THE INVENTION

The present invention is generally directed to a speech discrimination testing system, for testing the speech comprehension ability of a person using a speech discrimination system.

### BACKGROUND OF THE INVENTION

Hearing tests are commonly used to evaluate the sensitivity of a person's hearing and the ability of a person to comprehend speech. There are many types of hearing tests.

Pure Tone Audiometry is one of the more common hearing tests used, and measures the ability of a person to hear pure tone sound frequencies, typically between 250Hz to 8,000Hz. The test applies to both air and bone conduction audiometry, and may therefore be used to identify various causes of hearing loss.

Evaluation of candidacy for cochlear implantation typically involves consideration of both the degree of hearing threshold elevation (i.e. testing the minimum sound level of a pure tone that the subject can hear - Pure Tone Audiometry), and the accuracy with which a subject understands speech while wearing hearing aids, with the greater weight given to the latter.

The Hearing in Noise Test ('HINT') is another existing test, and measures a person's ability to hear speech in a noisy environment. During the HINT test, a subject is required to repeat sentences presented to them with: no competing noise, and with competing noise directed from various locations around the subject. The test measures signal-to-noise ratio, formed by the level of the sentences relative to the noise, needed for the subject to correctly repeat 50% of the words in the sentences.

There are several shortcomings identified with the HINT test, and similar language-based hearing tests. In order for the subject to correctly repeat sentences partially masked by noise, the subject must be capable of understanding the sentences played. This in turn requires the subject to be highly familiar with the language and phrases from which the presented sentences are derived. The test may therefore require variation to suit the native language of the subject (i.e. the test must be presented in a different language). This in turn affects the inherent difficulty of the test for different subjects, since certain languages, phrases and words may be easier to comprehend above noise than others (i.e. since the test is not exactly the same for different subjects, the difficulty of the test is subject to variation). Also, a physician may be required to carry out the test. Despite the availability of speech recognition software, the physician may still be required to assess the subject's ability to repeat sentences heard above noise. An additional shortcoming is that performance on a sentence test also depends on the subject's ability to use syntactic knowledge (i.e. grammar) and semantic knowledge (i.e. the meaning of words) to make inferences about the identity of individual words that have been masked by the background noise, and on working memory ability. These abilities vary among people, hence affect the score on the test, yet do not necessarily relate to the physical hearing abilities of the subject.

Thus, even though it is widely recognised that speech comprehension testing has proven useful, particularly as an indicator of candidacy for cochlear implantation, speech comprehension testing methods have not been widely adopted in many countries. This has in turn affected the number or referrals for cochlear implant candidacy as now discussed.

Cochlear implants are medical devices which bypass the damaged inner ear to electrically stimulate auditory neurons for improving sound and speech perception in people with severe to profound hearing loss. Despite the substantial speech perception benefits provided by cochlear implantation, there is a significant under-referral of adult patients for cochlear implant candidacy evaluation. Previous research has suggested that less than 6% of adults who need or would benefit from cochlear implantation actually receive them, partly as a consequence of the lack of appropriate assessment tools to identify those who require referrals for candidacy evaluation.

Returning to hearing tests generally, attempts have been made to address shortcomings in existing hearing tests, as now illustrated.

US patent 7288071 B2 discloses an automated hearing test system capable of performing several tests, including for speech discrimination. In the speech discrimination test described, the subject is audibly presented with words and required to select from words either graphically represented in writing or as a picture. For example, the subject may be audibly presented with the word 'horse' and then asked to select the image of a horse from a selection of other images. The disclosed test eliminates the need for a hearing professional, such as a physician, to either perform or attend the test. However, the disclosed test does not address any of the language dependent issues identified above. In particular, a different test for each language that may be spoken by subjects is still required, the relative difficulty of the test may still be affected by the choice of language, and the test may be affected by the subject's ability to use semantic knowledge.

US patent 8844358 B2 discloses a method of performing a hearing test on-line. According to the test proposed, meaningless syllables, called 'logatomes', are presented to a test person in fluctuating interference noise. In particular, at least one meaningless syllable is presented to a subject and the subject is required to identify and select the meaningless syllable he or she has heard from a range of graphically presented meaningless syllables displayed on a monitor or similar device. The disclosed method presents several disadvantages. In particular, despite the presented syllable being 'meaningless', the subject is still required to recognise the sound and either:
(a) identify it when graphically represented (e.g. as letters or characters) amongst a selection of alternative graphically represented syllables; or
(b) reproduce the sound so that it may be registered by speech recognition.

US patent 9148732 B2 builds on the test disclosed in US 8844358 B2. To improve the accuracy or efficiency of the provided test, voice signals for presentation are dynamically selected taking account of the previous responses of the subject. The adaptive selection of voice signals allows voice signals to be presented that are neither much too difficult nor much too easy for the subject, and prevents having to present the user with the entire inventory of available meaningless syllables.

While US 8844358 and US 9148372 both similarly address issues identified with language specific hearing tests, shortcomings may be identified. In particular, the subject is still required to recognise and identify a presented speech sound. If the subject is required to verbally repeat a heard speech sound, the accuracy of the test is necessarily subj ect to accuracy of speech recognition software, and the ability of speech recognition software to recognise speech of different users with varying first languages and accents. If the test requires the subject to identify and select a visual written signal, the test cannot quickly and simply be applied to any language, since languages necessarily use different writing systems around the world. For example, while the English language may be commonly written using a Latin alphabet, Russian may be written in Cyrillic, and Japanese may be written in kanji and kana. Further, languages utilising a similar writing style may differ in the pronunciation of particular characters. For example, the letter J in Spanish and Serbo-Croation may sound like the letters H and Y respectively in English. As a result, despite moving away from a test requiring recognition of language, the test proposed will still require review and modification to transfer between languages. Lastly, to identify and select different syllables, the test would require the subject to be able to read, or at least to be able to unambiguously associate sounds with symbols representing them.

US patent 9131876 B2 describes a method for determining whether a subject can identify syllables or phonemes. According to the method described, syllable or phoneme sound signals are presented to the subject in isolation, and the system is configured to determine whether each sound signal is audible. The sound signals are presented at several of their constituent frequency bands and the hearing characteristics of the subject are then determined by measuring the audibility of sound signals at those various frequency bands. The method uses the information obtained to determine the amount of amplification needed to make each sound audible, and does not test for the ability of the subj ect to identify them, not whether they can be discriminated from other sounds. As such, the test is not for example, suitable for use in identifying candidacy for cochlear implantation.

It would be advantageous, though not an essential object of the invention described, to provide a method or device for assessing the ability of a person to hear speech sounds which:
tests the ability of a person to hear speech sounds in a language-neutral manner;
does not require a hearing professional to be present during testing; or
can be used to estimate whether a person would be likely to hear speech sounds better if they used a cochlear implant.

Finlay discloses "Teaching heart auscultation to health professionals" in: "Teaching heart auscultation to health professionals", 31 December 2011. Finley teaches how methods of training speech discrimination can be applied to training of heart beat discrimination, i.e. teaching heart auscultation.

US 9 148 732 B2 relates to a method for testing hearing aids by presenting meaningless syllables with an audible voice signal and selecting the heard meaningless syllable from the displayed meaningless syllables by the user.

US 2017/273602 A1 relates to a system that enables an audiologist to relatively easily define and/or use any one or more of a variety of hearing tests for testing a subject's hearing.

The above discussion of background art is included to explain the context of the present invention. It is not to be taken as an admission that the background art was known or part of the common general knowledge at the priority date of any one of the claims of the specification.

### SUMMARY OF THE INVENTION

The invention is defined by the claims . According to a first aspect of the invention, there is provided a speech discrimination testing system according to claim 1.

In an embodiment, the at least one transducer comprises a loudspeaker.

In an embodiment, the speech discrimination testing system further comprises a display for presenting visual images to the subject, and the method further comprises:
presenting visual images to the subject in association with presented speech sounds; and
requiring the subject to identify which speech sound in the presented sequence of speech sounds was the lone speech sound, by identifying the visual image associated with that speech sound.

In an embodiment, the step of presenting visual images to the subject comprises presenting a sequence of visual images synchronised with the presentation of speech sounds such that each presented speech sound is associated with a presented visual image.

In an embodiment, the display comprises any one or more of the following selection: an electronic visual display, a television, a computer monitor, a touch screen, or a projector and projector screen.

In an embodiment, the display comprises a touchscreen and the subject's identification of the lone speech sound is inputted by touching a visual image presented on the touch screen.

In an embodiment, the method is performed by the subject utilising the speech discrimination testing system without input or assistance from another person.

Speech sounds stored in the inventory of speech sounds are used in the majority of the most commonly spoken languages.

In an embodiment, speech sounds stored in the inventory of speech sounds and selected for presentation follow a vowel-consonant-vowel format, a consonant-vowel-consonant format, a consonant-vowel format, or a vowel-consonant format.

In an embodiment, vowels used in the speech sounds are selected from a group consisting of: [a], [i] and [o]; and the consonants used in the speech sounds are selected from a group consisting of: [j], [k], [l], [m], [n], [p], [t] and [s].

In an embodiment, the speech sounds presented in a sequence vary from one another by substitution of either one vowel, or one consonant.

In an embodiment, speech sounds are presented within a sequence as a consonant pair.

In an embodiment, more than one sequence of speech sounds is presented such that the subject is required to identify lone speech sounds within each presented sequence.

In an embodiment, the method further comprises emitting noise of any type, including random noise, Brownian noise, or competing speech signal or signals, via the at least one transducer while the speech sounds are presented, to provide a signal-to noise-ratio such that the subject is required to discriminate between presented speech sounds while the noise is emitted.

In an embodiment, the signal-to-noise ratio at which speech sounds are presented against emitted noise is adjusted while speech sounds are presented to the subj ect.

In an embodiment, the signal-to-noise ratio is adjusted from sequence to sequence to account for inherent difficulty in discriminating speech sounds presented in each sequence.

In an embodiment, the signal-to-noise ratio is adjusted from sequence to sequence to ensure that each sequence provides substantially the same likelihood of identifying the correct lone speech sound.

In an embodiment, the signal to noise ratio is adjusted from sequence to sequence to ensure that the subject has a likelihood in the range of about 60% to about 80%, preferably about 70%, of identifying the correct lone speech sound for each presented sequence.

In an embodiment, the signal-to-noise ratio is adjusted based on responses received from the subject, to identify a signal-to-noise ratio at which the subject may correctly discriminate speech sounds at a pre-determined ratio of correct responses to incorrect responses.

In an embodiment, the level at which the speech sounds and, if applicable, noise are presented is adjusted to prevent background noise from affecting test performance.

In an embodiment, the method further comprises estimating whether, or the likelihood that, the subject's test results would be improved through cochlear implantation.

In an embodiment, the estimation of whether, or the likelihood that, the subject's test results would be improved through cochlear implantation is calculated in view of how long the subject has experienced hearing loss.

In an embodiment, results or analyses of the test are automatically sent to a hearing professional or the person being tested following completion of the test.

According to the invention, there is provided a speech discrimination testing system according to claim 1.

In an embodiment, the at least one transducer comprises a loudspeaker.

In an embodiment, the system further comprises a display for presenting visual images to the subject, and wherein the speech discrimination testing system is further configured to
present visual images to the subject in association with presented speech sounds; and
enable the subject to identify which speech sound in the presented sequence of speech sounds was the lone speech sound, by identifying the visual image associated with that speech sound.

In an embodiment, the speech discrimination testing system is configured to present visual images to the subject in a sequence of visual images synchronised with the presentation of speech sounds such that each presented speech sound is associated with a presented visual image.

In an embodiment, the display comprises any one or more of the following selection: an electronic visual display, a television, a computer monitor, a touch screen, or a projector and projector screen.

In an embodiment, the display comprises a touchscreen and the speech discrimination testing system is configured to enable input of the subject's identification of the lone speech sound by touching a visual image presented on the touch screen.

In an embodiment, the speech discrimination testing system is configured to enable the subject to complete a speech comprehension test without input or assistance from another person.

Speech sounds stored in the inventory of speech sounds are used in the majority of the most commonly spoken languages.

In an embodiment, speech sounds stored in the inventory of speech sounds follow a vowel-consonant-vowel format, a consonant-vowel-consonant format, a consonant-vowel format, or a vowel-consonant format.

In an embodiment, vowels used in the speech sounds are selected from a group consisting of: [a], [i] and [o]; and the consonants used in the speech sounds are selected from a group consisting of: [j], [k], [l], [m], [n], [p], [t] and [s].

In an embodiment, the speech discrimination testing system is configured to present speech sounds in a sequence such that the presented speech sounds vary from one another by substitution of either one vowel, or one consonant.

In an embodiment, the speech discrimination testing system is configured to present speech sounds within a sequence as a consonant pair.

In an embodiment, the speech discrimination testing system is configured to present more than one sequence of speech sounds such that the subject is required to identify lone speech sounds within in each presented sequence.

In an embodiment, the speech discrimination testing system is configured to emit noise via the at least one transducer while the speech sounds are presented, to provide a signal-to noise-ratio such that the subject is required to discriminate between presented speech sounds while the noise is emitted.

In an embodiment, the speech discrimination testing system is configured to adjust the signal-to-noise ratio at which speech sounds are presented against emitted noise while speech sounds are presented to the subject.

In an embodiment, the speech discrimination testing system is considered to adjust the signal-to-noise ratio from sequence to sequence to account for inherent difficulty in discriminating speech sounds presented in each sequence.

In an embodiment, the speech discrimination testing system is configured to adjust the signal-to-noise ratio such that all sequences presented have approximately the same likelihood of being correctly discriminated, when averaged across a representative group of subjects.

In an embodiment, the speech discrimination testing system is configured to adjust the signal-to-noise ratio based on responses received from the subject, to identify a signal-to-noise ratio at which the subject may correctly discriminate speech sounds at a pre-determined ratio of correct responses to incorrect responses.

In an embodiment, the speech discrimination testing system is configured to adjust the level at which the speech sounds and, if applicable, noise to the subject to minimise the effect that background noise has on the subject's test performance.

In an embodiment, the system is configured to estimate whether, or the likelihood that, the subject's test results would be improved through cochlear implantation following testing.

In an embodiment, the system is configured to estimate whether, or the likelihood that, the subject's test results would be improved through cochlear implantation taking into account how long the subject has experienced hearing loss.

In an embodiment, the speech discrimination testing system is configured to automatically send results to a hearing professional or the person being tested following completion of the test by a subject.

Throughout this specification and the claims which follow, unless the context requires otherwise, the word "comprise" and variations thereof such as "comprises" and "comprising", will be understood to include the inclusion of a stated integer or step or group of integers or steps but not the exclusion of any other integer or step or groups of integers or steps.

### BRIEF DESCRIPTION OF THE DRAWINGS

It will be beneficial to further describe the invention with respect to the accompanying drawings, which demonstrate a preferred embodiment of a device for testing the ability to discriminate speech sounds. Other embodiments of the invention are possible, and consequently, the particularity of the accompanying drawings is not to be understood as superseding the generality of the preceding description of the invention.
Figure 1 shows a phonetic inventory matrix in respect of vowel sounds used in the forty most common languages.
Figure 2 shows a phonetic inventory matrix in respect of consonant sounds used in the forty most common languages.
Figure 3 is a continuation of Figure 2 and shows a phonetic inventory matrix in respect of consonant noises used in the forty most common languages.
Figure 4 shows a speech discrimination testing system according to an embodiment of the invention.
Figure 5 illustrates a speech discrimination testing system according to certain embodiments of the invention in block form.
Figure 6 shows a screen shot of a visual representation associated with a first speech sound according to an embodiment of the invention.
Figure 7 shows a screen shot of a visual representation associated with a second speech sound according to an embodiment of the invention.
Figure 8 shows a screen shot of a visual representation associated with a third speech sound, inviting the subject to select a which visual representation is associated with a lone speech sound.
Figure 9 shows a flow-chart demonstrating working of a speech discrimination testing system according to an embodiment of the invention.
Figure 10 shows, for subjects wearing cochlear implants selected for phase I testing, the resulting overall test scores, expressed as a proportion correct responses as a function of signal-to-noise ratio.
Figure 11 shows, for subjects wearing cochlear implants selected for phase I testing, the resulting mean scores per consonant pair, expressed as proportion correct as a function of signal-to-noise ratio, prior to the signal-to-noise ratio being adjusted to different values for each consonant pair.
Figure 12 shows, for subjects wearing cochlear implants selected for phase I testing, the effect of vowel context on resulting overall scores.
Figure 13 shows, for subjects selected for phase II testing and using hearing aids alone, the distribution of hearing levels, expressed as four-frequency average hearing loss.
Figure 14 shows, for subjects selected for phase II testing and using hearing aids, the distribution of mean overall scores for speech discrimination testing according to an embodiment of the invention.
Figure 15 shows, for subjects selected for phase II testing and using cochlear implants, the distribution of mean overall scores for speech discrimination testing according to an embodiment of the invention.
Figure 16 shows the estimated probability of scoring higher in LIT with CIs for different LIT scores with HAs. The four rows depict different values for duration of hearing loss.
Figure 17 shows a flow chart of a main programme module controlling the general sequence of a speech discrimination test according to embodiments of the invention.
Figure 18 shows a flow chart of a module controlling a speech discrimination test according to embodiments of the invention.

### DETAILED DESCRIPTION OF THE INVENTION

In an embodiment the inventors have provided a test based on the ability of a subject to discriminate between speech sounds, rather than on a subject's ability to identify a speech sound, thereby allowing for a test method and device that may be effectively applied across language barriers. Such a test may never require the subject to identify a speech sound (such as by spelling it out or identifying it as a written word or a word of a language), other than to discriminate which sound is different to another that has been presented.

In an embodiment the invention is based on measuring the ability of a subject to correctly discriminate the odd speech sound present in sequence of other speech sounds. For example, pairs of speech sounds may be presented in sequential triplets, in which a first speech sound is presented once and a second speech sound is presented twice (such that the first speech sound is the `odd one out'). For example, if two speech sounds presented are [aja] and [ata], the subject may be presented with a sequential triplet of: [aja], [aja], [ata]. The subject would then be required to specify which of the three speech sounds was different to the other two. In this way, the subject is not required to identify either sound but is only required to recognise that one is different to the other. In other embodiments the speech sounds need not be presented as triplets, but rather as quadruplets, quintuplets, etc., and more than two different speech sounds may be presented.

### Speech sounds for presentation to a subject

In embodiments of the invention non-word speech sounds may be used as stimuli for a speech discrimination test so as to minimise the undue influence of language and subject learning on the ability of a subject to characterise a sound, and otherwise maximise the language neutrality of the test. This is because, if the presented speech sounds are commonly words of a particular language, then speakers of that language would have the advantage of being familiar with, and hence better able to discriminate those words over other words, phrases or speech sounds that they are less familiar with.

Nonsense speech sounds of various forms may be used according to embodiments of the invention. Nonsense speech sounds may in an embodiment take a consonant-vowel-consonant [CVC] format or a vowel-consonant-vowel [VCV] format. In an embodiment vowel-consonant-vowel [VCV] sounds may be used to maximise the availability of acoustic cues for discrimination by the subject, through perception of the medial consonant.

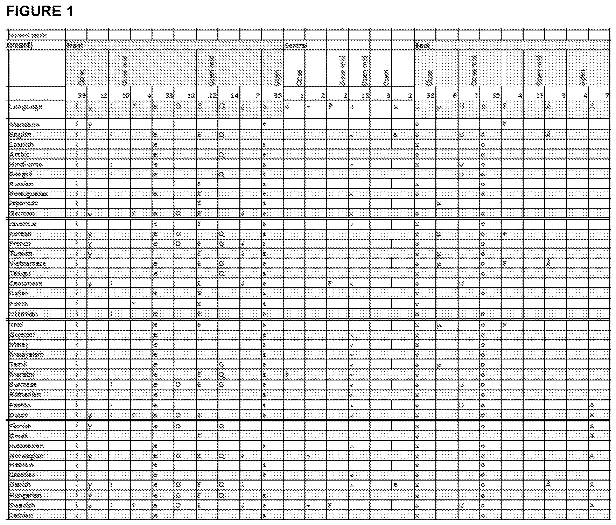

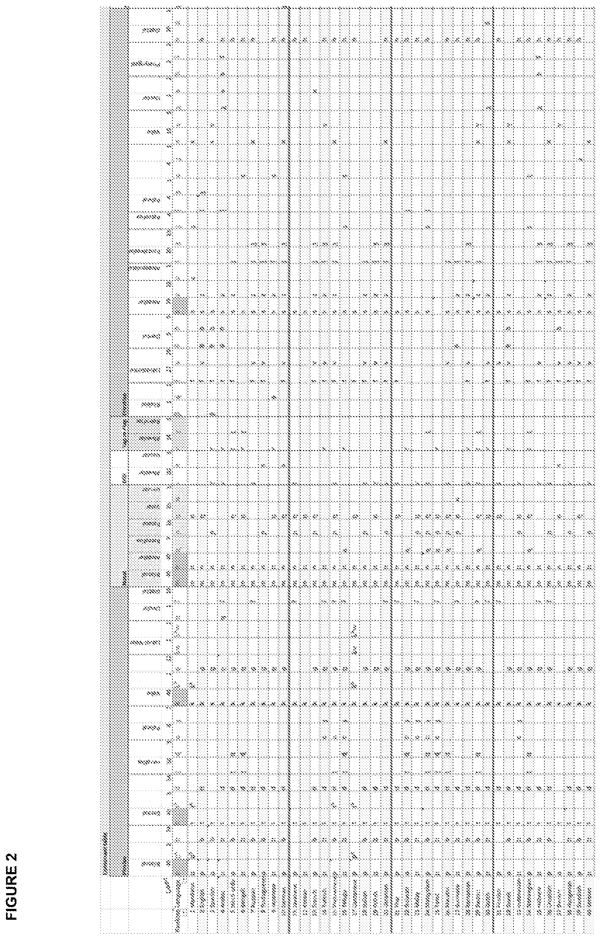

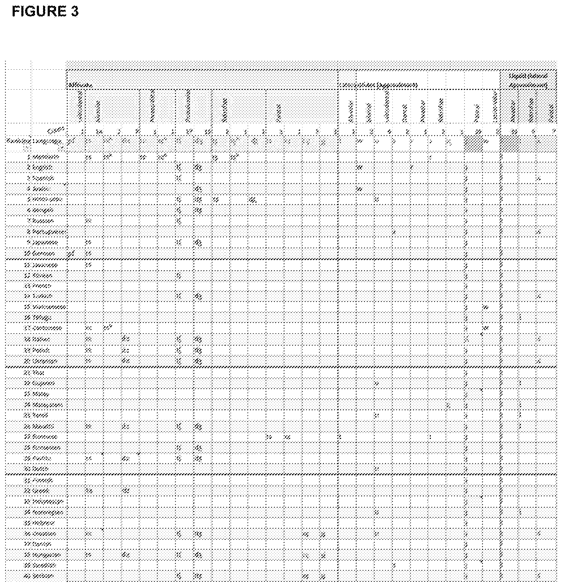

As part of their development of the invention, the inventors conducted a literature review on phonemic inventories occurring in the most widely spoken languages, being Mandarin, English, Spanish, Arabic, Hindi-urdu, Bengali, Russian, Portuguese, Japanese, German, Javanese, Korean, French, Turkish, Vietnamese, Telugu, Cantonese, Italian, Polish, Ukranian, Thai, Gujarati, Malay, Malayalam, Tamil, Marathi, Burmese, Romanian, Pashto, Dutch, Finnish, Greek, Indonesian, Norwegian, Hebrew, Croatian, Danish, Hungarian, Swedish, and Serbian. This was followed by a literature review on confusion matrices in consonant identification tasks, in noise and in quiet, for adult listeners: having typical hearing, using hearing aids, and using cochlear implants. To demonstrate the process undertaken by the inventors, a phonetic inventory matrix for the above languages respect of vowel sounds is included in Figure 1, while Figure 2 and Figure 3 in combination provide a phonetic inventory matrix in respect of consonant sounds. "The most commonly spoken languages" is herein defined to mean the 40 languages listed in this paragraph.

Consonants or vowels which are not common to a popular spoken language, and thereby may not be readily distinguishable by a transducer of that popular language, may be avoided for presentation according to certain embodiments of the invention. For example, speakers of the Hindi language are known to commonly have difficulty discriminating between [v] and [w] sounds, since the two are allophones in that language. Thus, in certain embodiments of the invention, [v] and [w] sounds may be avoided for presentation.

Accordingly, in certain embodiments of the invention, consonants for presentation to a subject may be reduced to a shortlist which avoids consonants that may not be readily distinguishable by speakers of each the most common languages. Such a shortlist may consist of the consonants [p] [t] [k] [m] [n] [s] [l] and [j]. In an embodiment these consonants may in turn be combined with the vowels [a], [i] and [o] to form [CVC] or [VCV] speech sounds which are believed to be generally distinguishable by speakers of the most commonly spoken languages.

Pursuant to the above, it is believed that speakers of the most commonly spoken languages should generally be able to readily discriminate between each of the following speech sounds: [apa], [ata], [aka], [ama], [ana], [asa], [ala], [aja], [ipi], [iti], [iki], [imi], [ini], [isi], [ili], [iji], [opo], [oto], [oko], [omo], [ono], [oso], [olo], and [ojo]. In an embodiment, speech sounds are presented in [VCV] format with the same vowel used twice in a given speech sound. Further, when presenting a pair of speech sounds in a sequence to a subject, a pair of speech sounds presented may share the same vowel, but differ in the selection of a consonant, so as to provide a `consonant pair' of speech sounds presented to the subject. Following this embodiment, suitable speech sound pairs, or consonant pairs, would for example include: [asa] and [ala], or [imi] and [isi]. Presenting the above exemplified [VCV] speech sounds in consonant pairs allows for 84 different combinations of consonant pairs for presentation to a subject in accordance with this embodiment.

In a further embodiment, speech sounds presented together in a sequence need not utilise the same vowel or consonant twice. Speech sounds such as [ato] and [ito], or [ima] and [omi] may therefore be used together, as well as speech sound such as [tom], [tok], [pot] and [son]. In further embodiments, speech sounds need not be presented as consonant pairs. In broader embodiments any speech sounds may be used as part of the test to enable a subject to discriminate between speech sounds.

### Hearing analysis method and system

In certain embodiments enabling self-administration, a hearing discrimination test may be implemented via a system comprising: a desktop or laptop computer, a tablet computer such as an iPad, a smart phone, a home assistant device such as a `Google Home', or another device or combination of devices as further exemplified below. In each of the devices exemplified above, the device comprises a data processor and a memory in data communication with the data processor. In certain embodiments, as further described below, the memory may comprise a hard drive device contained inside or outside a particular device. In other embodiments, the memory may be provided remotely such as via the 'cloud'. Other embodiments are also described below.

Speech sounds may be presented in sequential triplets, or longer sequences, from a transducer such as a loudspeaker integral to or separate from a device such as those described above. In an embodiment, speech sounds may be presented via headphones, which may be connected via cable, Bluetooth, Wi-Fi or other avenue to another device. In another embodiment the speech sounds may be presented via a larger loudspeaker such as desktop speaker or home stereo system. In another embodiment speech sounds may be presented to the user directly via a hearing aid. In another embodiment, speech sounds may be presented to the user via a bone conductor transducer or other suitable form of sound transducer. Any device which is capable of presenting speech sounds to the user may be employed. Upon presentation of the speech sounds, the subject is presented with options to select which speech sound within a presented sequence is different from those others presented, as further described below.

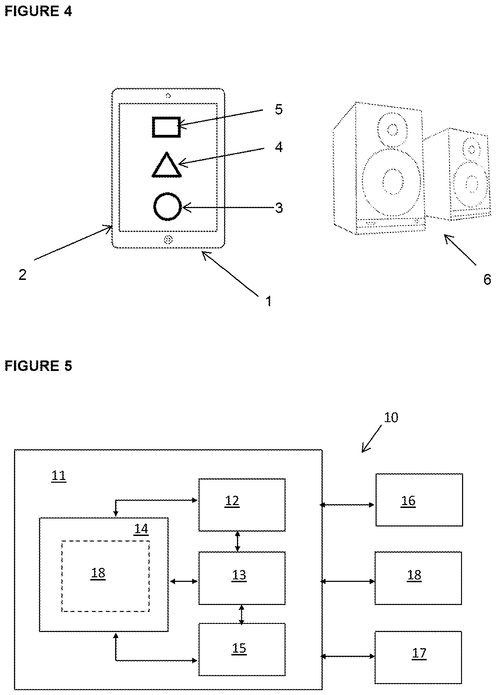

Figure 4 shows a system for administering a speech discrimination test according to an embodiment of the invention. As shown the system comprises a tablet computer device such as an iPad (1) and, in certain embodiments, a separate desktop loudspeaker (6) which may be connected via Bluetooth to the tablet computer (1). A speaker integral to the tablet computer (1) may be alternatively used. Speech sounds are presented to the subject (not shown) through the speaker (6) while visual representations (3), (4), (5) are presented on a screen (2) found on the tablet computer (1). Each visual representation (3), (4), (5) is associated with a presented speech sound, enabling the person to select a visual representation believed by the subject to be associated with a 'lone' speech sound in a given sequence of speech sounds. In the embodiment shown the screen (2) comprises a touchscreen which enables the subject to select a visual representation by touching the touchscreen. The screen (2) can also present the subject with results and analyses of testing, as appropriate. In other embodiments the subject may be presented with results and analyses by other methods, including via email.

Figure 5 illustrates a system (10) according to certain embodiments of the invention in block form. A computer (11) according to an embodiment of the invention has a number of functional components, including a video unit (12), a data processor (13), a memory unit (14) and a sound unit (15). In general, the video unit (12) provides the video signals that are displayed as images on the display screen (16) while the sound unit (15) provides the sound signals to the transducer (17). In some embodiments, the video unit (12) may be any one of several commercially available video cards and similarly the sound unit (15) may be any one of several commercially available sound cards. The data processor (13) is responsible for the overall operation of the computer (11), including execution of the operating system and any software applications residing on the computer (11). In some embodiments, the data processor (13) may be any one of several commercially available microprocessors. The memory unit (14) provides long-term and temporary (i.e., caching) storage for the software and data that are used by the computer (11) and may include one or more of, for example, a hard drive, main memory, removable storage (e.g., CD-ROM, floppy disk, flash card), and the like. As described further below, the memory unit (14) may comprise devices for remote storage, such as via the cloud.

The memory unit (14) may in some embodiments also store a speech discrimination test (18). More specifically, the memory unit (14) may store a computer-readable version of the speech discrimination test (18) that can be executed by the computer (11). During execution, a portion of the speech discrimination test (18) may be temporarily loaded from, for example, the hard disk and into the main memory components of the memory unit (14). As noted elsewhere, in addition to the stand-alone arrangement described above, it is also possible to execute the speech discrimination test (18) from a network. For example, the speech discrimination test (18) may be stored on a server computer (not expressly shown) that is accessible to several client computers. This arrangement has an advantage in that updates to the speech discrimination test (18) may be quickly and easily implemented. Other environments for executing the speech discrimination test (18) may also be used without departing from the scope of the invention.

The source code for the speech discrimination test (18) may be written in any suitable programming language (e.g.: C, C++, BASIC, Java). In addition, the speech discrimination test (18) can be implemented using a number of different programming methodologies (e.g., top-down, object oriented).

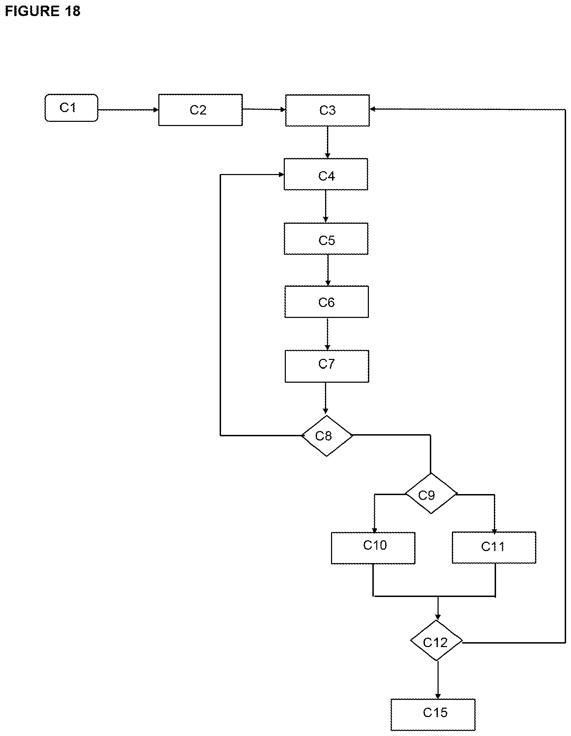

In one embodiment, the methodology of the speech discrimination test (18) involves a plurality of individual modules or object class modules with subroutines, properties and functions that can be called to perform specific tasks. The modules or subroutines can be called from a main routine, which would control the general sequence and flow of the speech discrimination test (18) and from within other modules or subroutines, which would control specific functions or tasks in either an isolated or cooperative manner. This is further exemplified below in respect of Figures 18 and 19 as discussed below.

Depending on particular embodiments, other components (16) of the system (10) that may be present include a keyboard, mouse or touchpad, microphone, printer and the like.

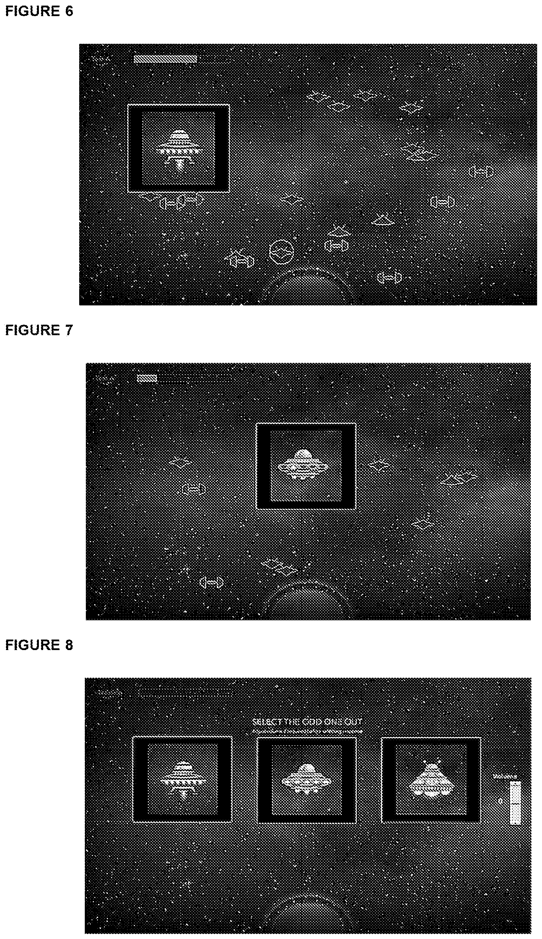

In Figures 6 to 8 visual images associated with speech sounds are presented as different spaceships so as to provide an interesting visual experience for the subject (and thereby maintain subject interest), though other visual images or devices which a subject may associate with a presented speech sound may of course be used. The spaceships appear in sequence as each speech sound is presented to the subject. For example, a first spaceship, as exemplified in Figure 6, may be presented when a first speech sound is presented. Thereafter a second spaceship, as exemplified in Figure 7, may appear when a second speech sound is presented, and so on. The subject thereby understands which spaceship is associated with a particular speech sound without requiring written or verbal explanation. Once all speech sounds are presented the subject is presented with all spaceships, as shown in Figure 8, and is required to select which spaceship is associated with the 'odd' speech sound. Upon making a correct selection, the subject may be presented with visual reward or similar to provide encouragement to continue the test.

Visual images may be presented to the subject via any suitable device or mechanism. In an embodiment, as described above, visual images may be presented via a touchscreen. This further enables the subject to select the 'odd' speech sound through interaction with the same touch screen. In other embodiments, visual images may be presented by for example a monitor, television, projector, or by another suitable mechanism such as a mechanical display (e.g. pop-up buttons). In other embodiments a screen might not be provided but, for example, a set of lights is provided which light up so that a given light, or colour of light, is associated with a given speech sound. In other embodiments a display may not be provided and the subject may simply be required to select speech sounds based on order, i.e. the subject may select the 'first' speech sound as the lone speech sound. Using this approach a blind subject may still have the ability to complete the speech discrimination test through either speech recognition software as further described below, by pressing on an associated `first sound' button, 'second sound' button or `third sound' button (based on the number of sounds presented) on a device; or by pressing a single button once, twice or three times as appropriate (again based on the number of sounds presented). A touch pad may for example be used as an alternative to a button.

In embodiments not involving a touchscreen a mechanism, component or device allowing the subject to select the 'odd' speech sound may be provided, such as a computer mouse, touchpad, keyboard, button arrangement, remote control, a motion detector (for example recognising a number of hand waves or particular facial movements) or sound detector (for example speech recognition software may allow the subject to simply state `first sound', 'second sound' or similar which would be recognised by the software). Using a voice recognition approach, and without requiring a display to provide for visual representations, other devices such as a home assistant (e.g. a `Google Home') or similar could be used to implement the test.

Where visual images are provided, rather than having a visual image appear as the speech sound is presented, in other embodiments the visual image may appear just before or just after the speech sound is presented, or the visual image may be highlighted in some way (such as by the visual image becoming larger, brighter, shimmering, moving, or changing colour), just before, during, or just after the speech sound is presented. Any mechanism which ensures that the subject can associate a presented visual image with a presented speech sound may be used according to embodiments of the invention.

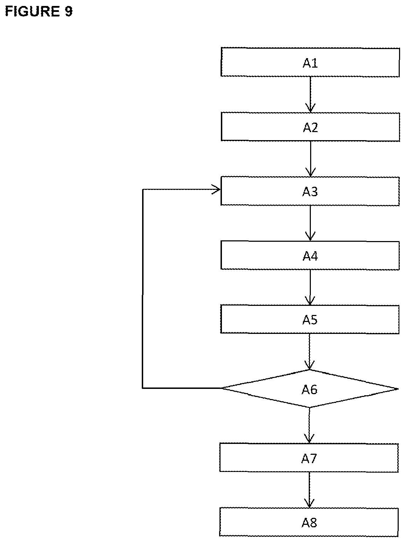

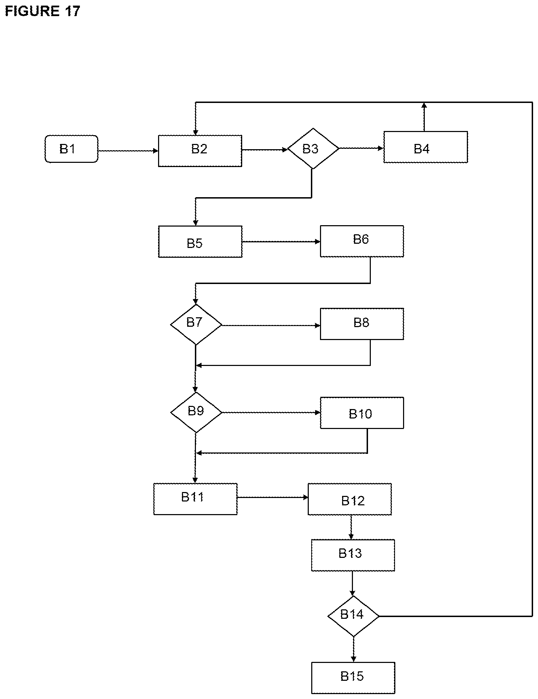

Figure 9 provides a general flow chart of steps involved in a hearing discrimination test according to an embodiment of the invention. At step A1 a hearing discrimination test system is presented to a subject. In accordance with certain embodiments of the invention, the hearing discrimination test system may comprise a tablet computer and desktop speaker as exemplified in Figure 4, or a system as exemplified in Figure 5. At step A2 the subject may initiate the hearing discrimination test by selecting an application on the tablet computer. At this stage the subject may enter other relevant information such as name, age, sex, years of hearing loss, and years of using a hearing aid or other relevant hearing device. At step A3 a first speech sound is presented to the subject in association with a first visual representation. At step A4 a second speech sound is presented to the subject in association with a second visual representation. At step A5 a third speech sound is presented to the subject in association with a third visual representation. The subject is then, at step A6, required to select which of the three presented speech sounds is different to the other two by selecting an appropriate visual representation. In this embodiment the subject may be presented with a triplet of speech sounds as a consonant pair, hence the presentation of three speech sounds. This sequence may be repeated as appropriate to provide sufficient test results for a given purpose. Once sufficient test results are provided data is analysed at step A7 to add up the subject's score and determine how well the subject can discriminate between speech sounds. In an embodiment, data may be analysed to determine the likelihood that the subject would obtain better test results upon, for example, cochlear implantation. At step A8 the subject is presented the results of any data analysis. In an embodiment the results may also be sent directly to a hearing professional for analysis to determine whether the subject is a candidate for cochlear implantation. Further detail on this process, provided according to embodiments of the invention, is provided in respect of Figure 17 and Figure 18.

### Development of a hearing discrimination test system and method - Stage I

A game-based method and system was developed to test the suitability of embodiments of the invention to test hearing and speech comprehension. In this game-based embodiment, speech sounds were selected to be presented as triplets of different [VCV] consonant pairs, and the method and device were designed to be self-administrable by adults.

Recordings of each speech sound were produced by a native Australian English female talker in an anechoic chamber. The recordings were reviewed by a panel, and two of the six recordings for each speech sound were selected for use in the test. All recordings were high-pass filtered at 250 Hz to minimise the impact of variations in frequency response at low frequencies for transducer outputs of a range of tablet computers and smartphones. The recordings were equalised in overall RMS level.

Preliminary testing was conducted with 50 subjects having hearing impairment. Criteria for subject selection involved hearing impaired adults: with at least one year of experience wearing cochlear implants, with no other co-morbidities affecting their communication ability, and who speak English as a first language. The subjects selected were users of either a single cochlear implant, a cochlear implant combined with a hearing aid, or bilateral cochlear implants. All subjects were tested when using a cochlear implant in one ear alone.

[VCV] speech sounds were presented during testing as consonant pairs (i.e. pairs sharing the same consonant but different vowels, e.g. [aka] and [ata]) at 65 dB SPL in speech-shaped noise. Signal-to-noise ratios were set for different presentation conditions: primarily Quiet, 7 dB signal to noise ratio ('SNR'), and 0 dB SNR, but at several other SNRs for some of the subjects.

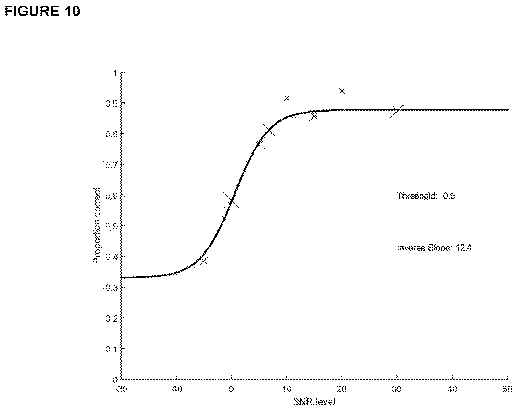

Figure 10 shows the overall scores, expressed as proportion correct, for all of the 50 subjects as a function of SNR. 'Quiet' scores are shown as scores at 30 dB SNR. A logistic function was fitted to the data using a maximum likelihood criterion. The higher asymptote demonstrates the stage at which improved SNR does not substantially improve the rate of correct answers from the 50 subjects. Similarly the lower asymptote demonstrates the stage at which poorer SNR does not affect the proportion of correct answers, which predictably provides a 33% correct answer rate given subjects were required to select between three multiple choice options as part of the test. The threshold value of 0.6 is the SNR at which the proportion correct is approximately halfway between the lower and upper asymptotes.

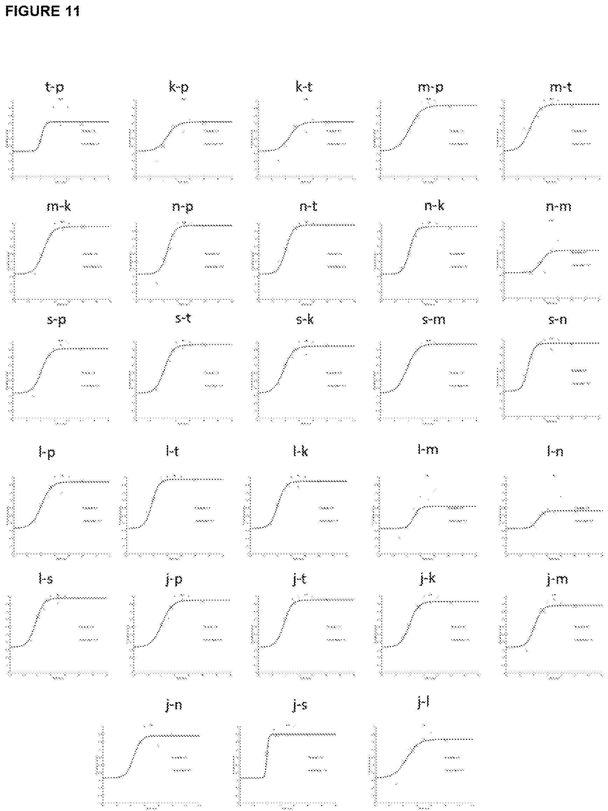

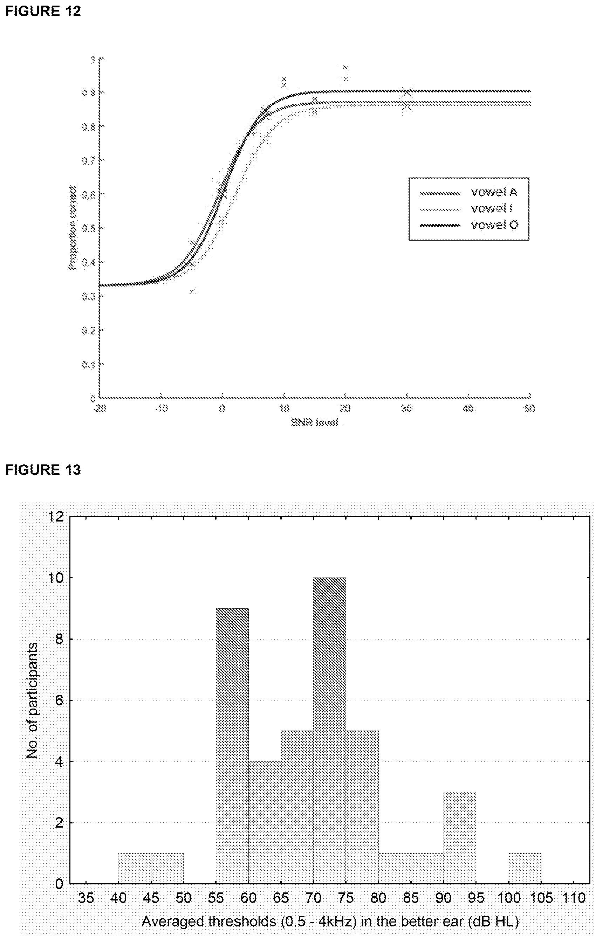

The mean scores per consonant pair (expressed as proportion correct as a function of signal-to-noise ratio) are shown in Figure 11. The effect of vowel context on overall scores is also shown in Figure 12. Results obtained from pilot testing were used to construct psychometric functions for each speech sound pair. Based on the results shown in these figures, a signal-to-noise ratio was specifically selected for each presented consonant pair such that, on average across the pilot subjects, each consonant pair was correctly discriminated by a subject 70% of the time. In other words, the inherent difficulty in discriminating between a particular consonant pair was balanced by adjusting the signal-to-noise ratio provided for that consonant pair (e.g. since [omo] and [ono] may be inherently difficult to discriminate, the SNR for that consonant pair is increased in comparison to another inherently more easy consonant pair to discriminate). As demonstrated by Figure 11, certain consonant pair speech sounds could be identified as having very low or very high performances, in terms of being inherently too hard or too easy to discriminate between. These were [iki] versus [iti]; [imi] versus [ini]; and

[ana] versus [ama]. For example, in respect of Figure 11, the n-m consonant pair is shown to provide a low proportion of correct answers even at high SNL ratios, demonstrating that this consonant pair provides high inherent difficulty to distinguish. These consonant pairs were removed for the purposes of further testing, leaving 81 available consonant pairs.

The reduced set of consonant pairs, each adjusted with their own sound-to-noise ratio, then became the set of stimuli to be used in the next stage of developing a speech comprehension testing method and device according to embodiments of the invention.

### Development and testing of a hearing analysis system and method - Stage II

A testing method and system implemented by the inventors on a laptop computer was evaluated to examine whether it would be suitable for objectively testing speech comprehension.

81 adult subjects participated in Phase II of the project. These included 41 adults wearing hearing aids with hearing loss of 4 frequency average hearing level (4FAHL) >40 dB HL, and 40 adults wearing 'Nucleus' cochlear implants with at least one year of cochlear implant experience. Table 1 gives the characteristics of participant subjects.

**Table 1 - characteristics of subjects**

| **Device** | **No. of participants** | **Age at Assessment (Yrs)** | **Age at first CI** | **Duration of use of first CI** |
|---|---|---|---|---|
| CI | 40 | Mean = 68.0 | Mean = 60.0 | Mean = 8.1 |
| | Unilateral = 7 | SD = 13.8 | SD = 18.1 | SD = 7.6 |
| | Bilateral = 10 | Median = 71.3 | Median = 62.7 | Median = 6.0 |
| | CI+HA = 23 | Range = (25.8 - 92.4) | Range = (4.8- 89.1) | Range = (1.1 - 32.0) |
| HA | 41 | Mean = 68.4 | | |
| | | SD = 12.3 | | |
| | | Median = 71.1 | | |
| | | Range = (35.7 - 89.8) | | |

For subjects using hearing aids alone, the distribution of hearing level, expressed as four-frequency average hearing loss ('4FAHL') across 0.5, 1, 2 and 4 kHz in the better ear is shown in Figure 13. The duration of hearing aid use ranged from 1 to 78 years. Of the 41 users of hearing aids alone at the time of testing, 38 were users of bilateral hearing aids (including 4 using `CROS' aids) and 3 were users of hearing aids in one ear only. Of those using unilateral hearing aids, two users had profound loss in one ear and a moderate-to-severe hearing loss in the other ear. The remaining one had bilateral moderate-to-severe hearing loss.

Prior to assessments, the hearing devices of the participants were checked. For users of hearing aids, otoscopy and tympanometry were performed to exclude cases of cerumen build up and/or middle ear dysfunction. Behavioural pure tone thresholds were measured in both ears using standard pure tone audiometry if an audiogram within 12 months of the test date was not available. Participants provided demographic information by completing a written questionnaire.

While subjects were wearing their devices (hearing aids or cochlear implants) at their personal settings, they were measured using a speech discrimination test (described as 'LIT' in several of the Figures) according an embodiment of the invention, using a laptop computer. The test was self-administered, with minimal input from an assessor. After written instructions were presented on screen, the subject was directed to adjust the overall loudness of presentation by moving a slider to set a comfortable loudness level. This was followed by a practice run after which the subject completed a test run. After every 20 trials of speech sound triplets, the subject could either take a brief break or press a button to continue testing until all 81 triplet sequences (as described above) were completed. The SNR for each triplet was adjusted in accordance with the findings for Stage I described above. Each subject completed two runs of 81 triplet sequences.

While not incorporated as part of the test discussed above, the background noise of the surrounding environment (e.g. household) may be monitored and factored into the volume at which the test is presented to the subject. For example, if the subject is taking the test at home, and there are high levels of background noise in the vicinity, a device such as a computer may monitor the noise levels and automatically adjust the level at which the test is presented, as opposed to having the subject manually adjust loudness levels for comfort.

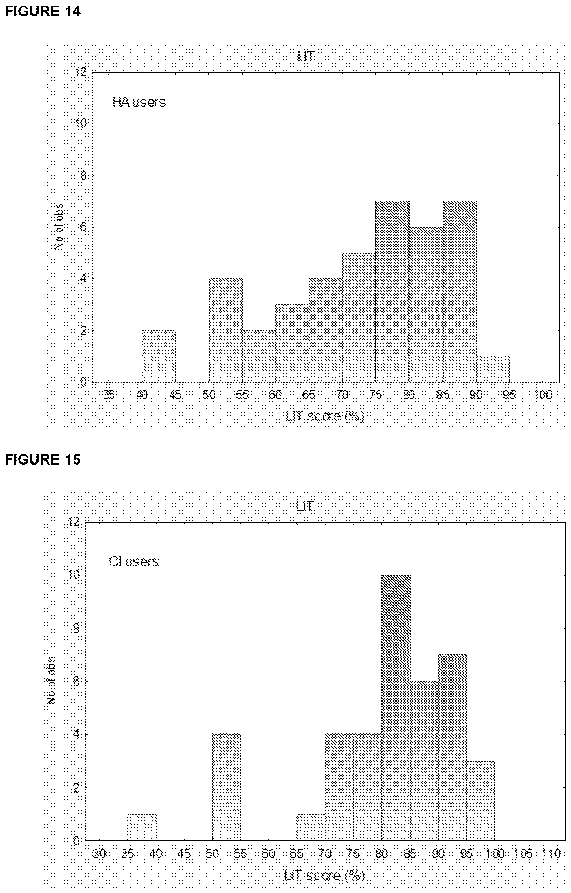

The below Table 2, and Figure 14, provide the distribution of mean overall scores for the Speech discrimination test, for hearing aid users.

**Table 2 - Summary scores of hearing aid users**

| | **Results (%)** |
|---|---|
| Mean | 72.4 |
| SD | 13.4 |
| 25^{th} percentile | 64.5 |
| 50^{th} percentile | 75.5 |
| 75^{th} percentile | 83 |
| Min-Max | 42 to 94 |

Similarly, the below Table 3, and Figure 15, provide the distribution of overall scores for the Speech discrimination test, for cochlear implant users.

**Table 3 - Summary scores of cochlear implant users**

| | **Results (%)** |
|---|---|
| Mean | 80.1 |
| SD | 14.1 |
| 25^{th} percentile | 75.8 |
| 50^{th} percentile | 82.8 |
| 75^{th} percentile | 90.0 |
| Min-Max | 36.5 to 99 |

As demonstrated by Table 2 and Table 3, cochlear implant users generally obtained better test results during speech discrimination testing, noting that the mean proportion of correct responses for hearing aid users was 72.4%, while cochlear implant users obtained a mean proportion of 80.1%.

While according to the embodiment analysed during Stage II testing, the metric extracted from subjects relates to the percentage of correct responses, it is nevertheless possible to utilise other metrics according to other embodiments of the invention. For example, by providing an adaptive signal-to-noise ratio during testing based on responses from the subject, it is possible to extract a signal-to-noise ratio at which a subject provides the correct response during the test at a pre-determined rate, such as for example 60%, 70%, or 80% of the time. In this way, a high signal to noise ratio would be indicative of poor speech discrimination abilities, since a high signal to noise ratio would be required to elicit a high proportion of correct responses. Alternatively, the signal-to-noise ratio need not be adjusted at all from sequence to sequence and in other embodiments it may be unnecessary to emit any noise at all during testing.

Product-Moment correlation analyses revealed large and highly significant correlations between the first and second runs of the speech discrimination test for users of cochlear implants (r = 0.907, p<0.001) and for users of hearing aids (r = 0.876, p<0.001). This indicated that the test used reliably differentiates between subjects with different degrees of ability to discriminate between speech sounds, so as to reliably identify those having limited ability to discriminate speech sounds.

### Using system and method to estimate benefits of cochlear implantation

The inventors performed analyses to determine whether a method and system for testing speech discrimination according to embodiments of the invention may be used to estimate whether the user of a hearing aid may benefit from cochlear implantation.

The results were used to adjust the test scores to remove the effect of duration of hearing loss. Then, the probability of a person using hearing aids to score higher with cochlear implants during testing was estimated as the proportion of cochlear implant users whose adjusted score was higher than the non-implanted person's adjusted score.

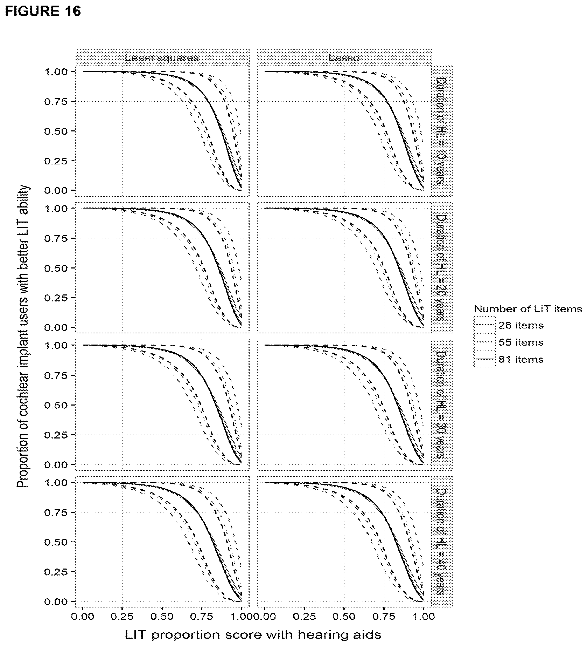

Further investigations on the impact by item-number on estimated probabilities were carried out using simulated data from the estimated distributions of performance with cochlear implants. Figure 16 shows the estimated probability of scoring higher in LIT with CIs for different LIT scores with HAs. The vertical axis provides the likelihood of improved testing while the horizontal axis provides the proportion of correct responses to the hearing discrimination test for hearing aid users. As generally shown, the lower the proportion of correct answers to the hearing discrimination test, the greater the likelihood that a hearing aid user would score better upon cochlear implantation. For example, a hearing aid user having 20 years of hearing loss, who correctly discriminated speech sounds 75% of the time, may be calculated to have a 78% chance of improved test results upon cochlear implantation. Further intuitively, a hearing aid user who correctly discriminated all speech sounds (i.e. 100% correct) would have a zero percent chance of improved test results upon cochlear implantation, as they could not perform any better on further testing.

The four rows depict different values for duration of hearing loss. The left panels depict estimates calculated using the least squares method, and the right panels depict estimates calculated using the lasso estimation method. Although Figure 16 shows estimates for LIT scores based on 28, 55 and 81 items in the test, all possible values for the total numbers of LIT items were considered. 95% confidence intervals are shown in dotted lines.

While development of certain embodiments of the software have been undertaken with having regard to determining whether a subject would obtain better test results upon receiving cochlear implantation, it is to be understood that applications of the invention are not so limited. More simply, the test may be used to test the speech discrimination or speech comprehension abilities of a subject. Other applications include the ongoing monitoring of the speech discrimination or speech comprehension skills of a subject through repeated testing to, for example, identify instances of hearing degradation, or improvement in speech discrimination skills following training in the use of a device. In another application, the test may be used by those who do not currently use a hearing aid, cochlear implant or similar so as to determine whether a person has poor speech comprehension or discrimination abilities and may benefit from use of a hearing device such as a hearing aid, or otherwise may benefit from visiting a hearing professional for further analysis.

### Program Modules

Having described the development of certain embodiments of the invention as set out above, now described with reference to Figure 17 is the operation of a main programme module controlling the general sequence of a speech discrimination test according to embodiments of the invention.

After initial power up, the main program module performs an equipment check at step B1 to ensure all components (for example transducers, screens,) of the system are functioning properly. Such a check may involve, for example, comparing the initial calibration data of the equipment with current measurements. In some embodiments, the various components of the equipment may be pre-calibrated together as a unit during manufacture or assembly. The calibration data may then be stored in a storage medium that is connected or attached to or sent together with the equipment. A determination is made at step B3 as to whether the equipment check passed, that is, whether the equipment is within a predetermined percentage of the initial calibration data. If the equipment check fails, then the main program module issues an equipment failure warning at step B4 and returns to the first step B1 to re-check the equipment.

If the equipment check passes, then the main program module proceeds to obtain the subject's information at step B5. This can be done, for example, by prompting the patient to manually enter his or her information (for example: name, address, date of birth, years of hearing difficulties, years of using a hearing aid, etc.), or by loading the information from a previously stored patient file. Here, as throughout the description, manual prompting may be done visually by displaying the instructions as text on a screen, or by audio instructions via a transducer, or by a combination of both. At step B6, the main program module allows the subject to select which test to be performed, for example a speech discrimination in which: no noise is emitted during testing (`Test A'), in which noise levels during testing are modified from sequence to sequence for ensure that presented consonant pairs are equally difficult to discriminate (`Test B'), or to modify noise levels during testing based on previous responses to as to identify an SNR at which the subject correctly identifies 'lone' speech sounds at a predetermined ratio (`Test C'). In the flow chart shown it is possible to select multiple tests to be performed one after the other. For simplicity, the flow chart exemplifies a main program module in which only options Test A and Test B are presented to the subject.

After the above selection, the main program module makes a determination as to whether Test A was selected at step B7. If Test A was selected, then at step B8, the main program module presents the subject with Test A according, in certain embodiments, to another module or sub-routine. If Test A was not selected, or otherwise upon completion of Test A, the main program module moves to step B9.

At step B9, the main program module makes a determination as to whether Test B was selected.. If Test B was selected, then at step B 10, the main program module presents the subject with Test B according, in certain embodiments, to another module or sub-routine. If Test B was not selected, or otherwise upon completion of Test B, the main program module moves to step B11.

At step B11, the main program module alerts the subject that he or she has completed the selected hearing tests. At step B12 the data processor may analyse data obtained testing, however this may alternatively be performed as part of the module or sub-routine of a completed test. At step B13 results and relevant analyses arising from the selected test(s) are presented to the subject and the subject is presented with a list of options at step B14 which includes: forwarding the results to a nearby hearing professional as step B15, printing the results as step B16, or undertaking a new test session, in which main program module returns the subject to step B2.

Now described with reference to Figure 18 is a flow chart is a module or sub-routine describing the general operation of a test according to an embodiment of the invention, selectable from a main program module as described with reference to Figure 17. In this example the module relates to a speech discrimination test in which noise levels during testing are modified from sequence to sequence for ensure that presented consonant pairs are equally difficult to discriminate (i.e. Test B identified above).

The test commences at step C1 in which the subject has selected Test B from the main program module. At step C2 suitable volume levels for performing the test are determined. This may be manually chosen by the subject upon being presented with sound and a choice to raise or lower presented sound so as to provide comfortable levels Otherwise it may be automatically selected by taking account of background noise levels as detected by a microphone where the test is being performed (so as to, for example, present the test at a predetermined level above background noise).

The test then commences at step C3 in which a selection is made of a consonant pair, and a noise / SNR level to present to the subject. This information may be stored in a memory of the system. The selection may be made randomly from stored consonant pairs, or in a set sequence. To avoid familiarity with the test, in an embodiment the selection is made randomly. At C4 a first speech sound is presented to the subject associated with a first visual representation. This step is repeated for a second speech sound and associated visual representation at C5, and for a third speech sound and associated visual representation at C6. At C7 the subject is presented with three visual representations and required to select which of the visual representations is associated with a 'lone' speech sound.

At C8 a time limit may be set on the amount of time provided to the subject to select a visual representation such that, if the subject does not respond in a given time, the subject is returned to step C4 so as to rehear the already-presented consonant pair triplet. In certain embodiments this step is removed since it provides the subject an opportunity to rehear a triplet that presented difficulties, which may not be desirable in certain embodiments. Otherwise, once the subject selects a visual representation the module is taken to step C9, where a determination is made as to whether the subject has correctly identified the visual representation associated with the lone speech sound. If the lone speech sound is correctly identified, it is added to the correct number count at C10. If the lone speech sound is not correctly identified, it is added to the incorrect number count at C11. In an embodiment, data pertaining to the consonant pair that obtained an incorrect response is stored to enable future improvement to the test (such as adjusting SNR levels for a given consonant pair if continued testing demonstrates that the paired speech sounds are more difficult to discriminate than previously understood).

Either way, following a correct response at C10 or an incorrect response at C11, the module is led to C12 in which a determination is made as to whether all consonant pair triplets have been presented to the subject. If not all consonant pair triplets have been presented to the subject, the module returns to C3 where a determination is made as to which consonant pair triplet to present to the subject next (while, in an embodiment, ensuring that consonant pair triplets are not repeatedly presented). If all consonant pair triplets have been presented then the test is stopped at C15 and the number of correct responses is recorded. In certain embodiments the recorded results may be retrieved later, which may be particularly useful where the test is applied to record ongoing speech discrimination skills of a subject through repeated testing over time.

### Summary

Based on the above, the inventors have developed a speech discrimination test which may, in an embodiment, be used to determine if a hearing-impaired subject is likely to obtain better test results upon cochlear implantation, as well as for other applications. In an embodiment, since the test may not require the subject to recognise speech sounds as words, a speech discrimination test may be language-neutral, as well as neutral regarding the syntactic and semantic knowledge of the subject. In an embodiment, speech sounds utilised in a speech discrimination test may be specifically selected to enable speakers of most languages to undergo the test.

In an embodiment, the test may be subj ect-implemented without necessitating the involvement of a hearing professional, such as a physician. Rather, the results of testing, in an embodiment including the likelihood that cochlear implantation would improve any further test results, may be automatically sent to a hearing professional and/or to the person taking the test, such as via email, for further consideration.

Modifications and variations as would be deemed obvious to the person skilled in the art are included within the ambit of the present invention as defined in the claims.

## Claims

1. A speech discrimination testing system (10), configured for testing the ability of a person to hear speech sounds in a language-neutral manner comprising:
a data processor (13);
a memory (14) in data communication with the data processor (13) configured to store an inventory of speech sounds for presentation to a subject; and
at least one transducer (17) for presentation of speech sounds to a subject wherein the speech discrimination testing system (10) is configured to:
select speech sounds from the stored inventory of speech sounds to be presented to the subject in a sequence;
present selected speech sounds via the at least one transducer to the subject in a sequence such that each speech sound in the sequence is presented to the subject more than once, apart from one lone speech sound which is presented to the subject only once;
input the subject's identification of the lone speech sound into the speech discrimination testing system (10);
**characterized in that**
speech sounds stored in the inventory of speech sounds are used in a majority of the most commonly spoken languages including Mandarin, English, Spanish, Arabic, Hindi-urdu, Bengali, Russian, Portuguese, Japanese, German, Javanese, Korean, French, Turkish, Vietnamese, Telugu, Cantonese, Italian, Polish, Ukranian, Thai, Gujarati, Malay, Malayalam, Tamil, Marathi, Burmese, Romanian, Pashto, Dutch, Finnish, Greek, Indonesian, Norwegian, Hebrew, Croatian, Danish, Hungarian, Swedish, and Serbian.

2. The speech discrimination testing system (10) according to claim 1, wherein the at least one transducer (17) comprises a loudspeaker (6).

3. The speech discrimination testing system (10) according to claim 1 or 2, wherein
the speech discrimination testing system (10) further comprising a display (16) for presenting visual images to the subject, wherein the speech discrimination testing system is further configured to present visual images to the subject in association with presented speech sounds; and
enable the subject to identify which speech sound in the presented sequence of speech sounds was the lone speech sound, by identifying the visual image associated with that speech sound.

4. The speech discrimination testing system according to claim 3, wherein the speech discrimination testing system is configured to present visual images to the subject in a sequence of visual images synchronized with the presentation of speech sounds such that each presented speech sound is associated with a presented visual image, and/or
wherein the display comprises any one or more of the following selection: an electronic visual display, a television, a computer monitor, a touch screen, or a projector and projector screen, wherein, optionally, the display comprises a touchscreen and the speech discrimination testing system is configured to enable input of the subject's identification of the lone speech sound by touching a visual image presented on the touch screen.

5. The speech discrimination testing system according to any one of the previous claims, wherein:
the subject utilizes the speech discrimination testing system without input or assistance from another person.

6. The speech discrimination testing system according to any one of the previous claims, wherein speech sounds stored in the inventory of speech sounds and selected for presentation to a follow a vowel-consonant-vowel format, a consonant-vowel-consonant format, a consonant-vowel format, or a vowel-consonant format,
wherein, optionally, vowels used in the speech sounds are selected from a group consisting of: [a], [i] and [o]; and the consonants used in the speech sounds are selected from a group consisting of: [j], [k], [l], [m], [n], [p], [t] and [s].

7. The speech discrimination testing system according to claim 5, wherein the speech sounds presented in a sequence vary from one another by substitution of either one vowel, or one consonant,
in which, optionally, speech sounds are presented within a sequence as a consonant pair.

8. The speech discrimination testing system according to any one of the previous claims, wherein more than one sequence of speech sounds is presented such that the subject is required to identify lone speech sounds within each presented sequence.

9. The speech discrimination testing system according to any one of the previous claims, further comprising emitting noise via the at least one transducer while the speech sounds are presented, to provide a signal -to noise-ratio such that the subject is required to discriminate between presented speech sounds while the noise is emitted.

10. The speech discrimination testing system according to claim 9, wherein the signal -to-noise ratio at which speech sounds are presented against emitted noise is adjusted during the test.

11. The speech discrimination testing system according to claim 10 when dependent on claim 5, wherein the signal-to-noise ratio is adjusted from sequence to sequence to account for inherent difficulty in discriminating speech sounds presented in each sequence, and, optionally, wherein the signal -to-noise ratio is adjusted from sequence to sequence to ensure that each sequence provides substantially the same likelihood of identifying the correct lone speech sound.

12. The speech discrimination testing system according to claim 11, wherein the signal to noise ratio is adjusted from sequence to sequence to ensure that the subject generally has a likelihood in the range of about 60% to about 80%, preferably about 70%, of identifying the correct lone speech sound for each presented sequence.

13. The speech discrimination testing system according to claim 10 or claim 11, wherein the signal-to-noise ratio is adjusted based on prior identifications received from the subject, to identify a signal-to-noise ratio at which the subject may correctly discriminate speech sounds at a pre-determined ratio of correct responses to incorrect responses.

14. The speech discrimination testing system according to any one of the previous claims, wherein the level at which the speech sounds and, if applicable, noise are presented is adjusted to prevent background noise from affecting test performance., and/or

15. The speech discrimination testing system according to claim 1, further comprising estimating whether, or a likelihood that, the subject's test results would be improved through cochlear implantation, and
estimating by taking into account how long the subject has experienced hearing loss.

16. The speech discrimination testing system according to any one of the previous claims, wherein results or analyses following the test are automatically sent to a hearing professional or the person being tested following completion of the test.

## Patentansprüche

1. Sprachunterscheidungstestsystem (10), konfiguriert zum Testen der Fähigkeit einer Person, Sprachlaute in einer sprachneutralen Weise zu hören, umfassend:
einen Datenprozessor (13);
einen Speicher (14) in Datenkommunikation mit dem Datenprozessor (13), der so konfiguriert ist, dass er einen Bestand von Sprachlauten zur Präsentation für eine Versuchsperson speichert; und
mindestens einen Wandler (17) zur Darbietung von Sprachlauten an eine Versuchsperson, wobei das Sprachunterscheidungstestsystem (10) so konfiguriert ist, dass es:
Sprachlaute aus dem gespeicherten Bestand von Sprachlauten, die der Versuchsperson in einer Sequenz präsentiert werden sollen, auswählt;
der Versuchsperson ausgewählte Sprachlaute über den mindestens einen Wandler in einer Folge präsentiert, so dass jeder Sprachlaut in der Folge der Versuchsperson mehr als einmal präsentiert wird, abgesehen von einem einzelnen Sprachlaut, der der Versuchsperson nur einmal präsentiert wird;
die Identifikation des einzelnen Sprachlauts durch die Versuchsperson in das Sprachunterscheidungstestsystem (10) eingibt;
**dadurch gekennzeichnet, dass**
die im Bestand der Sprachlaute gespeicherten Sprachlaute in den meisten der am häufigsten gesprochenen Sprachen verwendet werden, einschließlich Mandarin, Englisch, Spanisch, Arabisch, Hindi-urdu, Bengali, Russisch, Portugiesisch, Japanisch, Deutsch, Javanisch, Koreanisch, Französisch, Türkisch, Vietnamesisch, Telugu, Kantonesisch, Italienisch, Polnisch, Ukrainisch, Thailändisch, Gujarati, Malaiisch, Malayalam, Tamil, Marathi, Birmanisch, Rumänisch, Paschtu, Niederländisch, Finnisch, Griechisch, Indonesisch, Norwegisch, Hebräisch, Kroatisch, Dänisch, Ungarisch, Schwedisch und Serbisch.

2. Sprachunterscheidungstestsystem (10) nach Anspruch 1, wobei der mindestens eine Wandler (17) einen Lautsprecher (6) umfasst.

3. Sprachunterscheidungstestsystem (10) nach Anspruch 1 oder 2, wobei
das Sprachunterscheidungstestsystem (10) ferner eine Anzeige (16) umfasst, um der Versuchsperson visuelle Bilder zu präsentieren, wobei das Sprachunterscheidungstestsystem ferner so konfiguriert ist, dass es
der Versuchsperson visuelle Bilder in Verbindung mit präsentierten Sprachlauten präsentiert; und
die Versuchsperson in die Lage zu versetzen, zu erkennen, welcher Sprachlaut in der dargebotenen Folge von Sprachlauten der einzige Sprachlaut war, indem sie das diesem Sprachlaut zugeordnete visuelle Bild identifiziert.

4. Sprachunterscheidungstestsystem nach Anspruch 3, wobei das Sprachunterscheidungstestsystem so konfiguriert ist, dass es der Versuchsperson visuelle Bilder in einer Sequenz von visuellen Bildern präsentiert, die mit der Präsentation von Sprachlauten synchronisiert sind, so dass jeder präsentierte Sprachlaut mit einem präsentierten visuellen Bild assoziiert ist, und/oder
wobei die Anzeige eine oder mehrere der folgenden Auswahlmöglichkeiten umfasst: eine elektronische visuelle Anzeige, einen Fernseher, einen Computermonitor, einen Berührungsbildschirm oder einen Projektor und einen Projektorbildschirm, wobei die Anzeige optional einen Berührungsbildschirm umfasst und das Sprachunterscheidungstestsystem so konfiguriert ist, dass es die Eingabe der Identifikation des einzelnen Sprachlauts durch die Versuchsperson durch Berühren eines auf dem Berührungsbildschirm dargestellten visuellen Bildes ermöglicht.

5. Sprachunterscheidungstestsystem nach einem der vorhergehenden Ansprüche, wobei:
die Versuchsperson das Sprachunterscheidungstestsystem ohne Eingabe oder Unterstützung durch eine andere Person benutzt.

6. Sprachunterscheidungstestsystem nach einem der vorhergehenden Ansprüche, wobei Sprachlaute, die in dem Bestand von Sprachlauten gespeichert sind und zur Präsentation ausgewählt werden, einem Vokal-Konsonant-Vokal-Format, einem Konsonant-Vokal-Konsonant-Format, einem Konsonant-Vokal-Format oder einem Vokal-Konsonant-Format folgen,
wobei optional die in den Sprachlauten verwendeten Vokale aus einer Gruppe ausgewählt sind, die aus folgenden besteht: [a], [i] und [o]; und die in den Sprachlauten verwendeten Konsonanten aus einer Gruppe ausgewählt werden, die besteht aus: [j], [k], [l], [m], [n], [p], [t] und [s].

7. Sprachunterscheidungstestsystem nach Anspruch 5, bei dem sich die in einer Sequenz dargebotenen Sprachlaute durch Ersetzen entweder eines Vokals oder eines Konsonanten voneinander unterscheiden,
wobei optional Sprachlaute innerhalb einer Sequenz als Konsonantenpaar präsentiert werden.

8. Sprachunterscheidungstestsystem nach einem der vorhergehenden Ansprüche, wobei mehr als eine Sequenz von Sprachlauten präsentiert wird, so dass die Versuchsperson einzelne Sprachlaute innerhalb jeder präsentierten Sequenz identifizieren muss.

9. Sprachunterscheidungstestsystem nach einem der vorhergehenden Ansprüche, das ferner das Aussenden von Rauschen über den mindestens einen Wandler umfasst, während die Sprachlaute dargeboten werden, um ein solches Signal-zu-Rausch-Verhältnis bereitzustellen, dass die Versuchsperson zwischen dargebotenen Sprachlauten unterscheiden muss, während das Rauschen ausgesendet wird.

10. Sprachunterscheidungstestsystem nach Anspruch 9, wobei das Signal-Rausch-Verhältnis, bei dem die Sprachlaute gegen das emittierte Rauschen präsentiert werden, während des Tests eingestellt wird.

11. Sprachunterscheidungstestsystem nach Anspruch 10, wenn er von Anspruch 5 abhängt, wobei das Signal-Rausch-Verhältnis von Sequenz zu Sequenz angepasst wird, um die inhärente Schwierigkeit bei der Unterscheidung der in jeder Sequenz dargebotenen Sprachlaute zu berücksichtigen, und wobei das Signal-Rausch-Verhältnis optional von Sequenz zu Sequenz angepasst wird, um sicherzustellen, dass jede Sequenz im Wesentlichen die gleiche Wahrscheinlichkeit für die Identifizierung des richtigen einzelnen Sprachlauts bietet.

12. Sprachunterscheidungstestsystem nach Anspruch 11, wobei das Signal-Rausch-Verhältnis von Sequenz zu Sequenz eingestellt wird, um sicherzustellen, dass die Versuchsperson im Allgemeinen eine Wahrscheinlichkeit im Bereich von etwa 60 % bis etwa 80 %, vorzugsweise etwa 70 %, hat, den richtigen einzelnen Sprachlaut für jede dargebotene Sequenz zu erkennen.

13. Sprachunterscheidungstestsystem nach Anspruch 10 oder 11, wobei das Signal-Rausch-Verhältnis auf der Grundlage früherer, von der Versuchsperson erhaltener Identifizierungen eingestellt wird, um ein Signal-Rausch-Verhältnis zu ermitteln, bei dem die Versuchsperson Sprachlaute in einem vorgegebenen Verhältnis von richtigen Antworten zu falschen Antworten korrekt unterscheiden kann.

14. Sprachunterscheidungstestsystem nach einem der vorhergehenden Ansprüche, wobei der Pegel, mit dem die Sprachlaute und gegebenenfalls das Rauschen dargeboten werden, eingestellt wird, um zu verhindern, dass das Hintergrundrauschen die Testleistung beeinträchtigt.

15. Sprachunterscheidungstestsystem nach Anspruch 1, ferner umfassend die Abschätzung, ob oder wie wahrscheinlich es ist, dass die Testergebnisse der Versuchsperson durch eine Cochlea-Implantation verbessert werden würden, und
Abschätzung unter Berücksichtigung der Dauer des Hörverlustes der Versuchsperson.

16. Sprachunterscheidungstestsystem nach einem der vorhergehenden Ansprüche, wobei die Ergebnisse oder Analysen nach dem Test automatisch an einen Hörgeräteakustiker oder die getestete Person nach Abschluss des Tests gesendet werden.

## Revendications

1. Système de test de distinction vocale (10), configuré pour tester la capacité d'une personne à entendre des sons vocaux de manière neutre en termes de langue comprenant :
un processeur de données (13) ;
une mémoire (14) en communication de données avec le processeur de données (13) configurée pour stocker un inventaire de sons vocaux pour une présentation à un sujet ; et
au moins un transducteur (17) pour une présentation de sons vocaux à un sujet dans lequel le système de test de distinction vocale (10) est configuré pour :
sélectionner des sons vocaux dans l'inventaire stockés de sons vocaux à présenter au sujet selon une séquence ;
présenter des sons vocaux sélectionnés via l'au moins un transducteur au sujet selon une séquence de sorte que chaque son vocal dans la séquence soit présenté au sujet plus d'une fois, à l'exception d'un son vocal isolé qui est présenté au sujet une fois seulement ;
entrer une identification par le sujet du son vocal isolé dans le système de test de distinction vocale (10) ;
**caractérisé en ce que**
des sons vocaux stockés dans l'inventaire de sons vocaux sont utilisés dans une majorité des langues parlées les plus courantes incluant le mandarin, l'anglais, l'espagnol, l'arabe, l'hindi et l'ourdou, le bengali, le russe, le portugais, le japonais, l'allemand, le javanais, le coréen, le français, le turc, le vietnamien, le télougou, le cantonais, l'italien, le polonais, l'ukrainien, le thaïlandais, le gujarati, le malais, le malayalam, le tamil, le marathi, le birman, le roumain, le pashtou, le hollandais, le finnois, le grec, l'indonésien, le norvégien, l'hébreu, le croate, le danois, le hongrois, le suédois et le serbe.

2. Le système de test de distinction vocale (10) selon la revendication 1, dans lequel l'au moins un transducteur (17) comprend un haut-parleur (6).

3. Le système de test de distinction vocale (10) selon la revendication 1 ou 2, dans lequel
le système de test de distinction vocale (10) comprenant en outre un affichage (16) pour présenter des images visuelles au sujet, dans lequel le système de test de distinction vocale est configuré en outre pour présenter des images visuelles au sujet en association avec des sons vocaux présentés ; et
permettre au sujet d'identifier quel son vocal présent dans la séquence présentée de sons vocaux était le son vocal isolé, en identifiant l'image visuelle associée à ce son vocal.

4. Le système de test de distinction vocale selon la revendication 3, dans lequel le système de distinction vocale est configuré pour présenter des images visuelles au sujet dans une séquence d'images visuelles synchronisées avec la présentation de sons vocaux de sorte que chaque son vocal présenté est associé à une image visuelle présentée, et/ou
dans lequel l'affichage comprend une quelconque d'un ou plusieurs de la sélection suivante : un affichage visuel électronique, une télévision, un moniteur d'ordinateur, un écran tactile, ou un projecteur et un écran de projecteur, dans lequel, facultativement, l'affichage comprend un écran tactile et le système de test de distinction vocale est configuré pour permettre une entrée d'identification par l'utilisateur du son vocal isolé en touchant une image visuelle présentée sur l'écran tactile.

5. Le système de test de distinction vocale selon l'une quelconque des précédentes revendications, dans lequel :
le sujet utilise le système de test de distinction vocale sans entrée, ni assistance d'une autre personne.

6. Le système de test de distinction vocale selon l'une quelconque des précédentes revendications, dans lequel des sons vocaux stockés dans l'inventaire de sons vocaux et sélectionnés pour une présentation dans un format qui suit un format voyelle-consonne-voyelle, un format consonne-voyelle-consonne, un format consonne-voyelle ou un format voyelle-consonne,
dans lequel, facultativement, des voyelles utilisées dans les sons vocaux sont sélectionnées à partir d'un groupe constitué de : [a], [i] et [o] ; et les consonnes utilisées dans les sons vocaux sont sélectionnées à partir d'un groupe constitué de : [j], [k], [l], [m], [n], [p] et [s].

7. Le système de test de distinction vocale selon la revendication 5, dans lequel les sons vocaux présentés dans une séquence varient l'un par rapport à l'autre par substitution soit d'une voyelle, soit d'une consonne,
dans lequel, facultativement, des sons vocaux sont présentés au sein d'une séquence sous forme d'une paire de consonnes.

8. Le système de test de distinction vocale selon l'une quelconque des précédentes revendications, dans lequel plus d'une séquence de sons vocaux sont présentées de sorte que le sujet soit tenu d'identifier des sons vocaux isolés au sein de chaque séquence présentée.

9. Le système de test de distinction vocale selon l'une quelconque des précédentes revendications, comprenant en outre une émission de bruit via l'au moins un transducteur tandis que les sons vocaux sont présentés, pour fournir un rapport signal-bruit de sorte que le sujet soit tenu de distinguer des sons vocaux présentés tandis que le bruit est émis.

10. Le système de test de distinction vocale selon la revendication 9, dans lequel le rapport signal-bruit auquel des sons vocaux sont présentés en opposition à un bruit émis est ajusté au cours du test.

11. Le système de test de distinction vocale selon la revendication 10, lorsque dépendant de la revendication 5, dans lequel le rapport signal-bruit est ajusté de séquence en séquence pour constituer une difficulté inhérente dans une distinction de sons vocaux présentés à chaque séquence, et, facultativement, dans lequel le rapport signal-bruit est ajusté de séquence en séquence pour garantir que chaque séquence fournit substantiellement la même probabilité d'identification du son vocal isolé correct.

12. Le système de test de distinction vocale selon la revendication 11, dans lequel le rapport signal-bruit est ajusté de séquence en séquence pour garantir que le sujet affiche généralement une probabilité dans la plage d'environ 60 % à environ 80 %, de préférence d'environ 70 %, d'identification du son vocal isolé correct pour chaque séquence présentée.

13. Le système de test de distinction vocale selon la revendication 10 ou la revendication 11, dans lequel le rapport signal-bruit est ajusté selon des identifications préalables reçues du sujet, pour identifier un rapport signal-bruit auquel le sujet peut distinguer correctement des sons vocaux selon un rapport prédéterminé réponses correctes/réponses incorrectes.

14. Le système de test de distinction vocale selon l'une quelconque des précédentes revendications, dans lequel le niveau auquel les sons vocaux et, si applicable, un bruit sont présentés est ajusté pour empêcher un bruit de fond d'affecter un déroulement de test, et/ou

15. Le système de test de distinction vocale selon la revendication 1, comprenant en outre une estimation que, ou une probabilité que, des résultats de test du sujet seraient améliorés via une implantation cochléaire, et une estimation en tenant compte de la durée sur laquelle le sujet a subi une perte auditive.

16. Le système de test de distinction vocale selon l'une quelconque des précédentes revendications, dans lequel des résultats ou des analyses consécutifs au test sont automatiquement envoyés à un professionnel de l'audition ou à la personne testée à la suite de la réalisation du test.
